# EUROPÄISCHE PATENTSCHRIFT

(11) **EP 0 967 986 B1**
(45) Veröffentlichungstag und Bekanntmachung des Hinweises auf die Patenterteilung: **09.10.2002**
(21) Anmeldenummer: 98906744.2
(22) Anmeldetag: 10.03.1998
(51) Int. Cl.: A61K 35/16, A61P 43/00

(54) **VERWENDUNG VON HUMANEM alpha 1-SAUREN GLYCOPROTEIN ZUR HERSTELLUNG EINER PHARMAZEUTISCHEN PRÄPARATION**
USE OF HUMAN alpha 1-ACID GLYCOPROTEIN FOR PRODUCING A PHARMACEUTICAL PREPARATION
UTILISATION D'alpha 1-GLYCOPROTEINE ACIDE HUMAINE POUR PRODUIRE UNE PREPARATION PHARMACEUTIQUE

(30) Priorität: 10.03.1997 AT 40997
(43) Veröffentlichungstag der Anmeldung: 05.01.2000
(73) Patentinhaber: Baxter Aktiengesellschaft, 1221 Wien (AT)
(72) Erfinder: PICHLER, Ludwig, A-1040 Wien (AT); MUCHITSCH, Eva-Maria, A-1140 Wien (AT); PHILAPITSCH, Anton, A-2490 Ebenfurt (AT); SCHWARZ, Hans-Peter, A-1180 Wien (AT); LINNAU, Yendra, A-1220 Wien (AT); TESCHNER, Wolfgang, A-1030 Wien (AT)
(74) Vertreter: Alge, Daniel, Mag. Dr. rer.nat.
(86) Internationale Anmeldenummer: AT9800060
(87) Internationale Veröffentlichungsnummer: WO98040087

(56) Entgegenhaltungen:
- HIROSHI MAEDA ET AL.: "Further characterisation of the effects of alpha-1-acid glycoprotein on the passage of human erythrocytes through micropores" CELL STRUCTURE AND FUNCTION, Bd. 9, Nr. 3, 1984, Seiten 279-290, XP002071328
- E.-M.MUCHITSCH ET AL.: "In vivo effect of alpha-1 acid glycoprotein on experimentally enhanced capillary permeability in guinea-pig skin" ARCHIVES INTERNATIONALES DE PHARMACODYNAMIE ET DE THERAPIE, Bd. 331, Nr. 3, 1996, Seiten 313-321, XP002071329
- CLAUDE LIBERT ET AL.: "Protection by alpha-1-acid glycoprotein against tumor necrosis factor-induced lethality" J.EXP.MED., Bd. 180, Nr. 4, 1994, Seiten 1571-1575, XP002071330
- HARUHIKO YAMADA: "The inhibitory effect of glutathione on platelet aggregarion and its application in experimental hemorrhagic shock" HIROSHIMA J. ANESTH., Bd. 20, Nr. 2, 1984, Seiten 101-111, XP002071331
- J.P.WILLIAMS ET AL.: "Alpha-1-acid glycoprotein reduces the local and remote consequences of reperfusion injury in the rat" FASEB JOURNAL, Bd. 10, Nr. 3, 1996, Seite a675 XP002071332
- K.STEHR: "Zur pathogenese und Therapie des Schocks im Kindesalter" KLIN.PÄDIAT., Bd. 188, Nr. 6, 1988, Seiten 479-488, XP002071333

## Beschreibung

Die Erfindung betrifft neue medizinische Verwendungen von Orosomucoid.

α₁ -saures Glycoprotein (AGP), welches auch Orosomucoid genannt wird, ist eine aus Plasma gewonnene Substanz, welche ein Molekulargewicht von 40 000 Dalton aufweist, und einen Kohlehydratanteil zwischen 30 und 50 % aufweist. AGP besteht aus einer einzigen Polypeptidkette mit 183 Aminosäuren und weist zwei Disulfidbrücken auf. Es umfaßt weiters fünf Kohlehydratketten, welche alle in der ersten Hälfte der Peptidkette lokalisiert sind. Diese Kohlehydratgruppen bestehen zu rund 14 % aus neutralen Hexosen, 14 % Hexosaminen, 11 % Sialsäure und 1 % Fructose. Je nach Quelle der AGP-Präparation bzw. Gewinnungs- oder Charakterisierungsmethode tritt AGP in unterschiedlichen Formen auf, welches sowohl auf Unterschiede in der Polypeptidkette als auch Unterschiede in der Kohlehydratkette zurückgeführt wird.

Die Eigenschaften und biologischen Funktionen von Orosomucoid sind in den Übersichtsartikeln von Schmid (in "The Plasma Proteins Structure Function and Genetic control", Vol. 1 (1975), Academic Press, Ed. Frank A. Putnam, 2nd Edition, Seiten 183-228) und Kremer et al. (Pharmacological Reviews 40 (1988), Seiten 1-47), beschrieben.

In der Medizin wurde AGP bislang als wesentliche Trägersubstanz für vorwiegend basische Medikamente in Plasma erachtet (siehe Kremer et al.).

Weiters konnte eine positive Wirkung von Orosomucoid bei Entzündungsreaktionen nachgewiesen werden. So beschrieben Denko et al. (Agents and Actions 15, 5/6 (1984), 539-540) eine antiinflammatorische Wirkung von AGP bei Uratkristallentzündungen in Ratten. Libert et al. (J. Exp. Med. 180 (1994), 1571-1575) wiesen nach, daß eine ähnliche Indikation in der Verhinderung von septischem Schock im Zusammenhang mit der Wirkung von TNF-α oder Lipopolysacchariden liegt.

Zur Verbesserung von Perfusionsstörungen, insbesondere der Mikrozirkulation, sowie Reperfusionsschäden sind bisher entweder vasoaktive Substanzen oder spezielle Blutfaktoren, die die Hämostase bzw. Fibrinolyse beeinflussen, insbesondere Antikoagulantien oder thrombolytisch wirksame Faktoren verabreicht worden, oder entsprechender Volumsersatz durchgeführt worden.

Die Gabe von Orosomucoid zur Behandlung eines mit TNF/Endotoxin induzierten Schocks wurde von Muchitsch et al. (Arch. int. Pharmacodyn. 331(3) (1996), 313-321) und Libert et al. (J. Exp. Med. 180(4) (1994), 1571-1575) beschrieben.

Zur Behandlung von hypovolämischen Schockzuständen, die unabhängig von Entzündungsreaktionen auftreten, erfolgte bislang Volumenersatz, wobei üblicherweise Albuminlösungen zum Einsatz kommen.

Aufgabe der vorliegenden Erfindung ist es, neue medizinische Indikationen für Orosomucoid zur Verfügung zu stellen.

Die vorliegende Erfindung betrifft daher die Verwendung von humanem α₁saurem Glycoprotein (Orosomucoid) zur Herstellung einer pharmazeutischen Präparation zur Behandlung von hämorrhagischem und/oder hypovolämischem Schock.

Überraschenderweise zeigte sich auch, daß Orosomucoid zur Behandlung von Störungen der Durchblutung bzw. der Mikrozirkulation nicht-inflammatorischer Art geeignet ist. Es kann daher zur Verbesserung von Perfusionsstörungen, insbesondere Störungen der Mikrozirkulation, sowie Reperfusionsschäden, und vor allem bei Schockzuständen zur besseren Versorgung der vitalen Organe, wie Hirn, Lunge, Herz, Leber und Niere, eingesetzt werden.

Diese Indikationen sind allesamt nicht-inflammatorischer Art, d.h., daß die Störungen dann indiziert sind, wenn sie nicht im Zusammenhang mit der SIRS ("systemic inflammatory response syndrom") auftreten. Zur Definition von SIRS siehe Critical Gare Medicine 20 ( 6 ) , 864-874 (1992). Bei Entzündungen werden nämlich Zellen und Gewebe direkt geschädigt, als Folge ist die Permeabilität der Gefäße und der Blutkreislauf gestört. Bei der erfindungsgemäßen Verwendung von AGP zur Herstellung einer pharmazeutischen Präparation zur Behandlung von hämorrhagischem und/oder hypovolämischem Schock sind diese Störungen jedoch nicht-inflammatorischer Art und sind somit durch andere Ursachen bedingt. Zu diesen Ursachen zählen geänderte Druckverhältnisse, vor allem im Zusammenhang mit der Verminderung des intravasalen Volumens. Die dadurch ausgelösten Durchblutungsstörungen führen im unbehandelten Fall zu hypovolämischem Schock. Als auslösende Mechanismen gelten dabei akute Blutungen, exzessive Flüssigkeitsverluste, wie Erbrechen, Durchfälle, extremes Schwitzen, Dehydratation, exzessiver Harnabsatz, Peritonitis, Pankreatitis, Ischämien im Splanchnikusgebiet, Darmverschluß, Gangrän, stumpfe Traumen, Beschädigung großer Muskelgruppen oder Verbrennungen.

Diese Zustände wurden bislang üblicherweise mit Dextranlösungen, Hydroxyethylstärke, Ringerlactat oder Albuminlösungen behandelt. Diese Substanzen weisen jedoch keinerlei anti-inflammatorische Eigenschaften auf, weshalb es überraschend war, daß Orosomucoid, welches für die Behandlung von Entzündungen eingesetzt wurde, in den erfindungsgemäßen Indikationen diese Funktion übernehmen konnte.

Orosomucoid kann auch bei relativer Hypovolämie verwendet werden. Diese liegt dann vor, wenn das absolute Blutvolumen nicht vermindert ist, jedoch eine Unterversorgung der Organe vorliegt. Der Grund kann eine vasodilatatorische Veränderung sein, die neurogen, metabolisch, toxisch oder humoral sein kann. Ein weiterer Grund ist eine gesteigerte Gefäßpermeabilität, eventuell anaphylaktischer Art oder bedingt durch diverse Schlangengifte.

Ebenso kann als Ursache für hypovolämischen Schock ein Pumpversagen sein, bedingt durch akuten Myokardinfarkt, Myocarditis oder eine stark verringerte Auswurfleistung, akute Klappeninsuffizienz, Myokardruptur, Septumperforation, Arrhythmien, wie Bradykardie, Tachykardie oder Fibrillation, bzw. durch mechanische Kompression des Herzens oder physikalische Hindernisse, beispielsweise Thromben oder Embolien.

Die vorliegende Erfindung betrifft daher die Verwendung von AGP zur Herstellung einer Präparation zur Behandlung von hämorrhagischem und/oder hypovolämischem Schock, insbesondere zur Stabilisierung des intravasalen Volumens, insbesondere bei akuten Blutungen, exzessivem Flüssigkeitsverlust bzw. Vasodilatation.

AGP kann dabei auch zur Verhinderung der Reperfusionsschäden als Folge eines Schlaganfalls, insbesondere zur Reduktion des Gehimödems eingesetzt werden. Reperfusionsschäden treten vor allem nach Beseitigung eines Strömungshindernisses, beispielsweise eines Gefäßverschlusses auf Grund von Ablagerungen oder Blutgerinnsel auf. Diese Schäden werden insbesondere als Folge eines Schlaganfalles beobachtet, wobei sich ein Gehirnödem bildet und es zu neurologischen Ausfällen kommt. Die Gewebeschäden in transplantierten Organen, welche aufgrund der wieder aufgenommenen Perfusion auftreten können, zählen ebenfalls zu den Reperfusionsschäden.

Eine weitere Störung der Mikrozirkulation, welche durch AGP behandelt werden kann, sind die Mikrozirkulationsstörungen in vitalen Organen, insbesondere in der Niere, welche beispielsweise unmittelbar eine Proteinurie bedingen können.

Die Mikrozirkulationsstörungen in den Organen können jedoch auch durch Ödeme bedingt sein. Daher kann Orosomucoid auch zur Herstellung einer Präparation zur Verhinderung bzw. Behandlung von Ödemen verwendet werden.

Die Herstellungsweise von AGP ist bekannt (beispielsweise wie aus der WO 95/07703) . Als Quelle für humanes AGP dient vorzugsweise humanes Plasma bzw. eine Plasmafraktion, beispielsweise eine COHN-Fraktion, wie COHN IV oder COHN V. Erfindungsgemäß wird die Präparation vorteilhafterweise als lagerstabile Infusionslösung hergestellt und bevorzugt als Lyophilisat zur Verfügung gestellt.

Die angewendete Dosis ist abhängig von der jeweiligen Indikation und auch der Schwere des Zustandes des Patienten, beispielsweise dem Ausmaß des Blutverlustes. In der Regel wird eine Einzeldosis im Bereich von 70 mg/kg Körpergewicht bis 5 g/kg verwendet, wobei der Bereich von 100 bis 700 mg/kg besonders bevorzugt ist.

Die erfindungsgemäße Applikation kann auf alle Arten erfolgen, bevorzugt sind dabei die i.v., s.c, i.m. und die lokale Applikation.

Vorteilhafterweise enthält die angewendete pharmazeutische Präparation mindestens 50 % Orosomucoid, vorzugsweise mehr als 70 %, insbesondere mehr als 90 %, bezogen auf das Gesamtprotein. Als weitere Komponente kann die erfindungsgemäße pharmazeutische Präparation weiters Albumin und A₁AT (α₁-Antitrypsin) enthalten.

Vorzugsweise wird der pharmazeutischen Präparation vor der erfindungsgemäßen Verwendung ein Stabilisator zugemischt, insbesondere Natriumcaprylat und gegebenenfalls Tenside, um die Lagerstabilität bzw. die Stabilität während einer Hitzebehandlung zu erhöhen.

Es ist auch zweckmäßig, die pharmazeutische Präparation zur Inaktivierung bzw. Abreicherung von Viren zu behandeln, insbesondere durch mindestens eine physikalische Behandlung, wie Hitzebehandlung und/oder Filtration. Zur Inaktivierung von Viren sind eine Reihe von physikalischen, chemischen oder chemisch-physikalischen Methoden bekannt, wie beispielsweise eine Hitzebehandlung, z.B. gemäß der EP 0 159 311 A oder der EP 0 637 451 A, eine Hydrolasebehandlung gemäß der EP 0 247 998 A oder eine Strahlenbehandlung oder eine Behandlung mit organischem Lösungsmittel und/oder Tensiden, z.B. gemäß der EP 0 131 740 A, umfassen. Weitere geeignete Virusinaktivierungsschritte bei der Herstellung der erfindungsgemäßen Präparate sind in der EP 0 506 651 A oder in der WO 94/13329A beschrieben.

Bei bestimmten Indikationen, nämlich insbesondere hypovolämischem oder neurogenem Schock, wird vorteilhafterweise die Verabreichung von Orosomucoid mit der Verabreichung von vasoaktiven Substanzen kombiniert (konstringierend oder dilatierend), welche entweder gemeinsam oder parallel verabreicht werden können.

Daher betrifft die vorliegende Erfindung gemäß einem weiteren Aspekt ein Infusionspräparat zur Behandlung von Schockzuständen, enthaltend als wirksame Bestandteile AGP und eine vasoaktive Substanz.

Erfindungsgemäß wird dieses Infusionspräparat in Form eines Sets zur Verfügung gestellt, welches
- AGP in einer pharmazeutischen Präparation und
- eine vasoaktive Substanz, gegebenenfalls in separaten Behältern,
umfaßt.

Die erfindungsgemäße Anwendung von AGP kann prophylaktisch, aber auch vor allem therapeutisch erfolgen.

Die Erfindung wird durch die nachfolgenden Beispiele und die Zeichnungsfiguren, auf welche sie jedoch nicht beschränkt sein soll, näher erläutert.

Es zeigen: Fig. 1 bis 5 die Ergebnisse der Behandlung von hämorrhagischem Schock im Rattenmodell; Fig. 6 bis 8 die Ergebnisse bei der Verhinderung des Gehirnödems im "Strokemodell" an der Ratte; und Fig. 9 die Ergebnisse der Behandlung der Proteinurie in einem Rattenmodell.

### Beispiel 1 : Behandlung von hämorrhagischem Schock im Rattenmodell (Derzeit nach Ansicht der Anmelderin der beste Weg zur Ausführung der Erfindung)

Diese Experimente wurden in analoger Weise zu Wang und Chaudry (J. Surg. Res 50 (1991), 163-169) durchgeführt. Die Tiere fasteten über Nacht, hatten aber freien Zugang zu Wasser. Um die Narkose einzuleiten, wurde den Tieren 60 mg/kg Pentobarbital i.m. injiziert. Die Narkose wurde durch 5 mg Pentobarbital pro Tier alle 1,5 Stunden s.c. aufrecht erhalten. Die Trachea wurde zur künstlichen Beatmung in Notfällen kanüliert. Ein Polyethylenkatheter wurde in die linke Jugularvene für Infusionen (Volumsersatz) und Injektionen vorgesehen. Ein zweiter Katheter wurde über die rechte Jugularvene in den rechten Vorhof zur Injektion von kalter isotonischer Kochsalzlösung (≤ 20°C; Thermodilutionsverfahren) eingeführt. Über die rechte Halsschlagader wurde ein Thermoelement in den Aortabogen zur Messung der Bluttemperatur vorgeschoben. Beide Oberschenkelarterien wurden kanüliert, eine für die Blutdruckbestimmung, die andere zum Blutentzug. Die Körpertemperatur wurde während des ganzen Experimentes unter Verwendung eines rektalen Thermometers, welches an eine Infrarotlampe angeschlossen war, auf 36,5°C gehalten. Um ein Trauma vor der Blutung zu setzen, wurde nach der Depilierung eine 5 cm Laparotomie in der Linea alba mittels eines Elektro-Kauters durchgeführt. Dieser Schnitt wurde danach schichtweise verschlossen.

1 IE Heparin/g Körpergewicht wurde injiziert. Anschließend wurde durch Blutentzug aus der Oberschenkelarterie innerhalb von 10 min der mittlere arterielle Blutdruck auf 40 mmHg gesenkt. Der Blutdruck wurde entweder durch weiteren Blutentzug oder durch Injektion von Ringer-Lösung in einem Gesamtvolumen nicht über 40 % des verlorenen Blutes für maximal 80 min bei 40 mmHg gehalten. Nach diesen 80 min oder früher (wenn der Blutdruck nicht mehr über 40 mmHg gehalten werden konnte) wurde mit der Völumssubstitution begonnen, indem das 3-fache Volumen des Gesamtblutverlustes durch Ringerlösung während 60 min ersetzt wurde. Der Volumssubstitution schloß sich eine Beobachtungsperiode von 4 h an. Die überlebenden Tiere wurden mit einer Überdosis von Pentobarbital i.v. getötet.

Der mittlere arterielle Blutdruck wurde fortlaufend mittels eines Polygraphen unter Verwendung eines elektromechahischen Druckwandlers registriert. Die Herzfrequenz wurde durch Pülswellen kontinuierlich aufgezeichnet. Das Herzminutenvolumen wurde mit der Thermodilutionsmethode bestimmt, wobei 200 µl kalte Kochsalzlösung in den rechten Vorhof injiziert wurden. Durch Messung der Bluttemperatur im Aortabogen wurde die Thermodilutionskurve mittels eines Cardiomax II (Model 85, Columbus Instruments) integriert und das Herzminutenvolumen in ml/min angegeben. Schlagvolumen und der gesamte periphere Gefäßwiderstand wurden berechnet, indem das Herzminutenvolumen durch die Herzfrequenz dividiert wurde bzw. indem der Blutdruck durch das Herzminutenvolumen dividiert wurde, wobei dieses Verhältnis mit 10³ multipliziert wurde (mmHg.ml.min⁻¹.10³). Initialwerte wurden als natürliche Werte angegeben. Alle anderen Werte wurden in % des jeweiligen Ausgangswertes (Δ%) angegeben. Mittelwerte ± Standardabweichung wurden ebenfalls berechnet. Die Signifikanz der Unterschiede zwischen Anfangswerten und allen anderen Werte wurden durch den t-Test für gepaarte Beobachtungen verifiziert. Zum Vergleich zwischen den Gruppen wurde der "zweiseitige t-Test" verwendet.

Es zeigte sich, daß bei allen Tieren (n = 30) die entzogene Blutmenge 7,0 ± 2,9 ml betrug, um den mittleren arteriellen Blutdruck auf 40 mmHg zu reduzieren. Der Blutdruck konnte während 77,8 ± 1,5 min auf diesem niedrigen Niveau gehalten werden. Der Blutdruckfall war begleitet von einem Absinken des Herzminutenvolumens, Schlagvolumens und des gesamten peripheren Gefäßwiderstandes. Die Herzfrequenz sank in allen drei Gruppen, wobei in der Gruppe, die mit AGP behandelt wurde, sich eine anfängliche Erhöhung eingestellt hatte.

Der Volumsersatz bei Kontrolltieren (n = 13) mit Ringerlösung i.v. (Volumen: 3-mal die Menge an verlorenem Blut) war nicht in der Lage, den mittleren arteriellen Blutdruck, der signifikant über den gesamten Beobachtungszeitraum gesunken war, wieder herzustellen (Bereich -50,5 ± 2,3 % bis -63,6 ± 10,3 %). Es konnte auch eine geringe Senkung der Herzfrequenz beobachtet werden, die 180 bis 240 min nach dem Volumsersatz signifikant wurde (Maximum: -29,9 ± 8,8 % bei 240 min). Das Herzminutenvolumen konnte unmittelbar nach dem Volumsersatz zu den Anfangswerten zurückgebracht werden, war aber zwischen 30 und 240 min danach signifikant gesenkt (Bereich -25,9 ± 5,5 % bis -51,4 ± 7,4 %). Der selbe Zeitverlauf konnte für das Schlagvolumen beobachtet werden (Bereich der Abnahme: -26,7 ± 8,8 % bis -31,1 ± 9,9 %). Der gesamte periphere Gefäßwiderstand sank während des Beobachtungszeitraums (Bereich -19,1 ± 18,2 % bis 39,7 ± 9,9 %), wobei die Unterschiede 30 bis 120 min nach der Reanimation signifikant waren. Drei Tiere starben ≤ 150 min nach dem Ersetzen des Volumens und wurden nicht in die Auswertung mit einbezogen. Drei weitere Tiere starben nach 180 min.

Zwei weitere Gruppen wurden unter Verwendung von AGP bzw. einer Placebo-Formulierung anstatt der Ringerlösung behandelt. Die AGP-Lösung (200 mg/kg), gereinigt aus COHN-Fraktion V von menschlichem Plasma durch Präzipitation und weitere Pasteurisierung bei 60°C für 10 h, und die analoge Menge an Placebo-Formulierung (Albuminlösung aus Humanalbumin; IMMUNO, durch Abtrennung von Orosomucoid) wurden mit Ringerlösung zur 3-fachen Menge des individuellen Blutverlustes verdünnt.

AGP wurde an 14 Tieren getestet; 1 Ratte starb während der Behandlung (Volumsersatz), 3 Ratten starben weniger als 150 min nach der Behandlung und 1 Tier starb nach 180 min nach dem Volumsersatz. Die Placebo-Formulierung wurde 18 Tieren verabreicht. Vier dieser Tiere starben während der Behandlung, vier starben weniger als 150 min nach der Behandlung, vier Tiere starben mehr als 180 min nach dem Volumsersatz. Ratten, welche vor 150 min nach der Behandlung starben, wurden bei der Auswertung nicht berücksichtigt.

Beim Vergleich der Resultate mit Ringerlösung mit denen der Placebo-Formulierung ergab sich, daß gleiche oder geringere Werte für mittleren arteriellen Blutdruck, Herzfrequenz und gesamten peripheren Gefäßwiderstand mit der Placebo-Formulierungsbehandlung erhalten wurden. Die Werte für Herzminutenvolumen und Schlagvolumen waren gleich oder höher in der Placeboformulierungsgruppe als in der Ringerlösung-Gruppe.

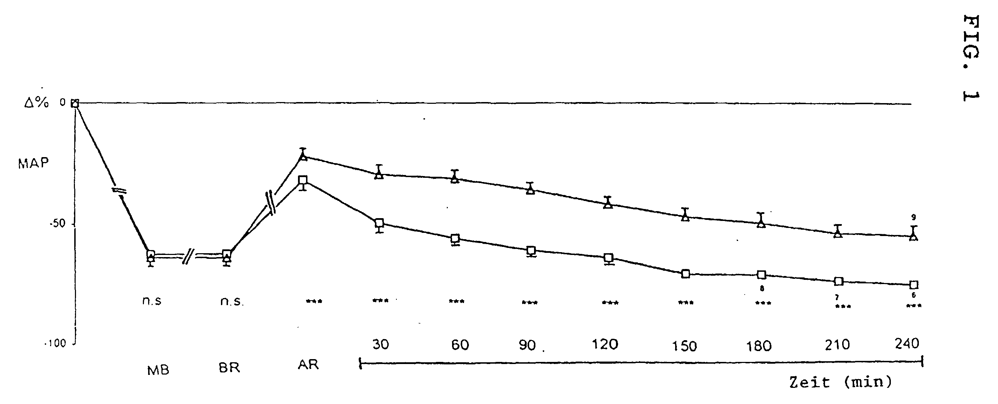

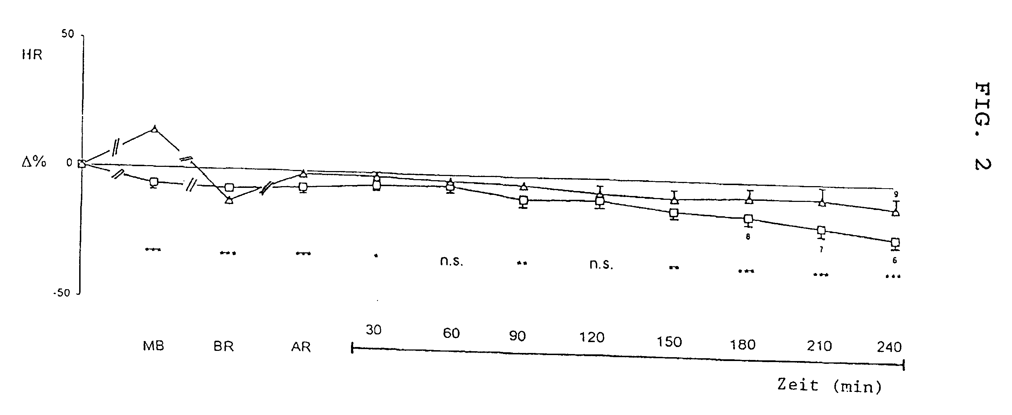

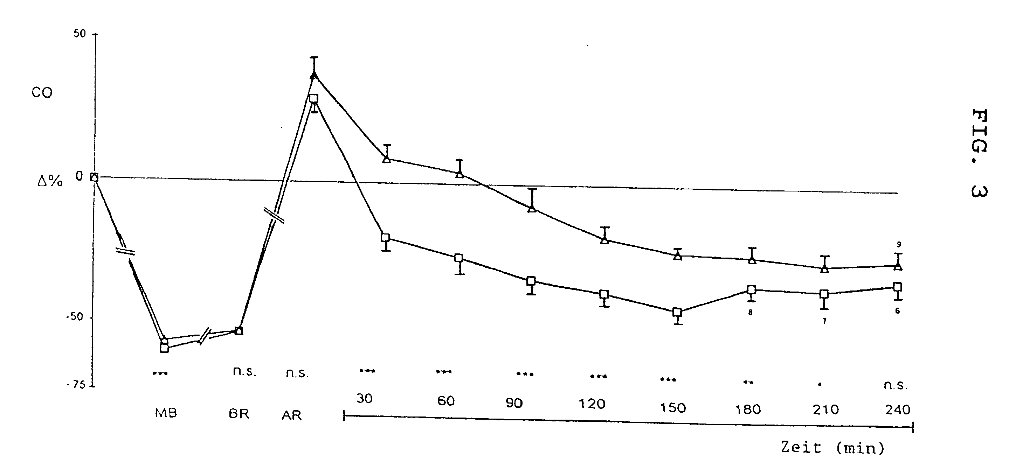

Bei Tieren, welche mit AGP behandelt wurden, stieg der Blutdruck anfangs und sank danach allmählich ab (siehe Fig. 1). Eine komplette Wiederherstellung des Blutdrucks konnte jedoch nicht erreicht werden. Alle Werte im Beobachtungszeitraum waren geringer als die Ausgangswerte (p ≤ 0,001). Bezüglich der Herzfrequenz (Fig. 2) konnte keine Änderung im Zeitraum nach der Volumensubstitution gegenüber den Ausgangswerten beobachtet werden (p > 0,05). Das Herzminutenvolumen (Fig. 3) war höher als die Ausgangswerte unmittelbar nach der Volumensubstitution (p < 0,01), stellte sich wieder auf die Ausgangswerte ein (+30 bis +90 min; p > 0,05) und sank schließlich unter die Ausgangswerte (p < 0,05 oder ≤ 0,01).

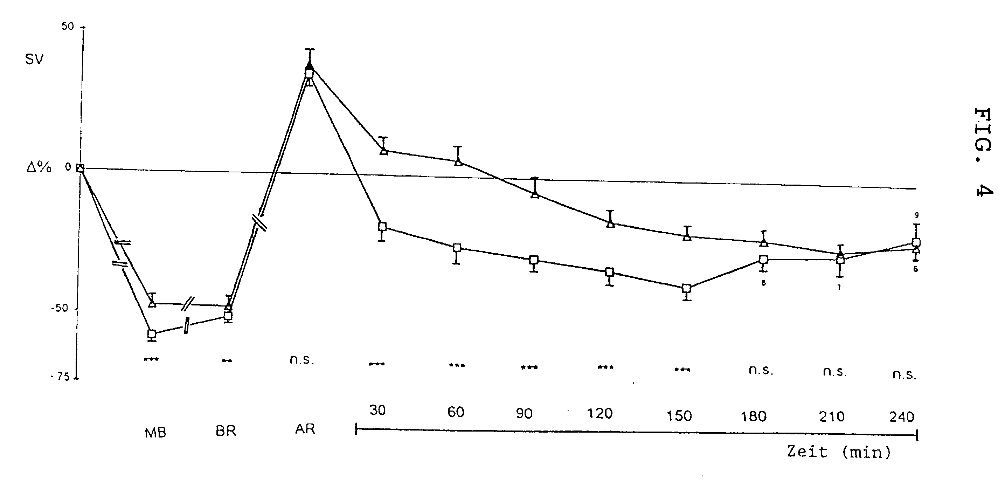

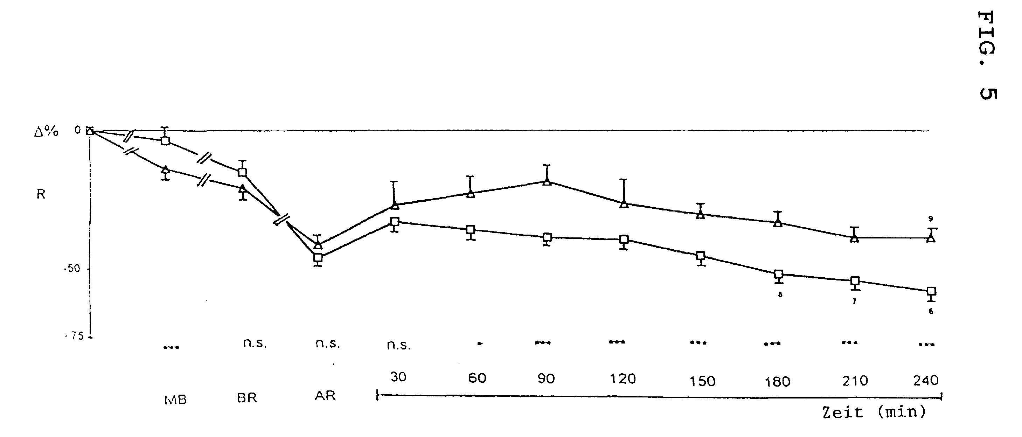

Eine ähnliche Situation ergab sich für das Schlagvolumen (Fig. 4) mit Ausnahme, daß die Werte während der ersten 120 min statistisch nicht unterschiedlich von den Ausgangswerten waren. Der gesamte periphere Gefäßwiderstand (Fig. 5) war während des gesamten Beobachtungszeitraumes niedriger-verglichen mit den Ausgangswerten; Signifikanz wurde jedoch bei +30, +90 und +120 min nicht erreicht.

Die Fig. 1 bis 5 zeigen auch den Vergleich zwischen Tieren, welche mit AGP und Placebo-Formulierung behandelt worden sind. Der mittlere arterielle Blutdruck ist bei allen Meßpunkten nach der Volumensubstitution in der mit AGP behandelten Gruppe signififikant höher (Fig. 1). Die Herzfrequenz ist gleich oder signifikant höher in der AGP-Gruppe, wobei die Unterschiede jedoch sehr klein waren (Fig. 2). Das Herzminutenvolumen ist in der mit AGP behandelten Gruppe signifikant höher, außer für den Meßpunkt "240 min" nach der Volumensubstitution (Fig. 3). Das Schlagvolumen ist nach der Behandlung mit AGP signifikant höher bei 30 bis 150 min nach der Volumensubstitution (Fig. 4). Der gesamte periphere Gefäßwiderstand ist in der AGP-Gruppe, verglichen mit der Placebo-Formulierung, bei 60 bis 120 min nach der Infusion erhöht (Fig. 5),

Diese Experimente zeigen die Überlegenheit einer Behandlung mit AGP im Vergleich mit einer Placebo-Formulierung (enthaltend dieselbe Proteinmenge in Form von Albumin, welches frei von AGP ist) oder Ringerlösung. Daraus leitet sich ab, daß AGP die Perfusion von lebenswichtigen Organen bei hypovolämischem Schock aufrecht erhalten kann.

### Beispiel 2 : Verhinderung des Gehirnödems im "Strokemodell" an der Ratte

Eine globale cerebrale Ischämie ("Stroke") wurde durch Abklemmen beider Halsschlagadern und dem Entzug von 5 ml Blut erreicht. Nach 30 min Ischämie wurden die Halsschlagadern wieder eröffnet und das entzogene Blut wieder infundiert. 23,5 Stunden später wurden die Tiere getötet und der Wassergehalt der beiden Großhirnhälften bestimmt.

In Vorversuchen wurde herausgefunden, daß Orosomucoid mit 600 mg/kg i.v. in der Lage ist, die Ausbildung eines Hirnödems nach globaler cerebraler Ischämie hintanzuhalten. Der Formulierungspuffer war ohne diesen Effekt geblieben. Im vorliegenden Beispiel wird eine Dosiswirkungs- und Zeitwirkungsbeziehung für den Ödem verhindernden Effekt von Orosomucoid erstellt.

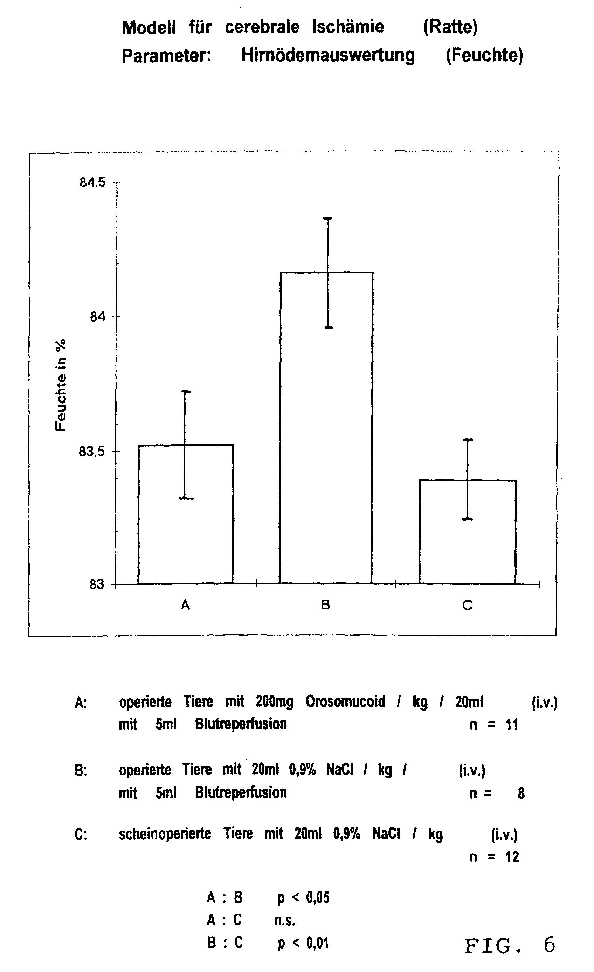

Orosomucoid, welches auch in Beispiel 1 verwendet wurde, wurde mit 200 mg/kg i.v. gleichzeitig mit der Blutreperfusion an Ratten geprüft. Die Ergebnisse gehen aus Fig. 6 hervor. Es zeigte sich, daß scheinoperierte Tiere (C, n = 12) kein Hirnödem haben, während ischämische, mit Kochsalzlösung behandelte Tiere ein massives Hirnödem aufweisen (B, n = 8). Ischämische Tiere, die mit Orosomucoid behandelt worden waren (A, n = 11), verhielten sich wieder wie scheinoperierte Tiere.

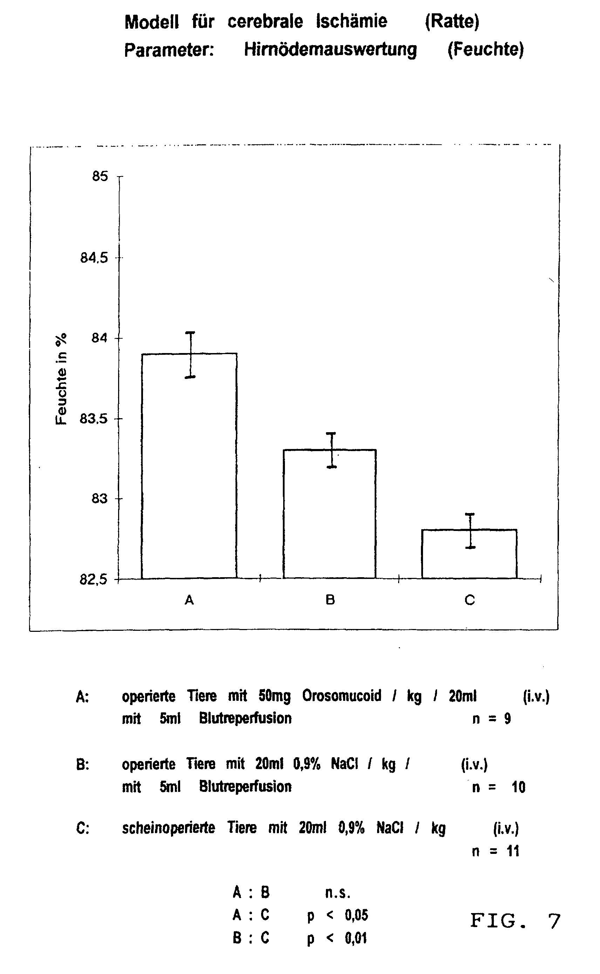

Bei einer Dosisreduktion auf 50 mg Orosomucoid pro kg i.v. konnte jedoch kein protektiver Effekt mehr festgestellt werden (siehe Säule A in Fig. 7), wodurch die Dosisabhängigkeit des Effekts belegt ist.

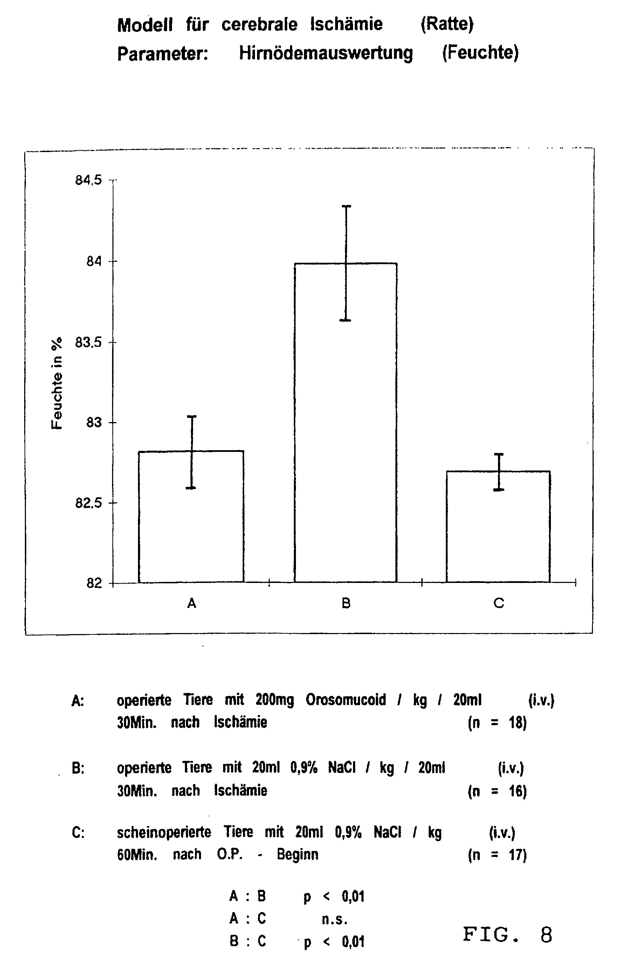

Auf Grund der therapeutischen Situation beim Menschen war es interessant zu prüfen, ob Orosomucoid auch nach eingetretenem Schlaganfall gegeben noch wirksam sei. Es wurde daher die oben als wirksam erkannte Dosis von 200 mg/kg i.v. 30 min nach Ischämieende verabreicht. Wie aus Fig. 8 hervorgeht (Säule A), ist Orosomucoid auch in dieser Situation voll wirksam.

Eine protektive Wirkung von Orosomucoid gegenüber dem im Gefolge eines Schlaganfalles entstehenden Hirnödem ist somit im Tierversuch bewiesen.

### Beispiel 3 : Behandlung der Proteinurie in einem Rattenmodell

Ratten wurden am Tag 0 mit 100 mg/kg Puromycin Aminonukleosid i.p. behandelt. Kontrollen erhielten in analoger Weise isotone Kochsalzlösung (negative Kontrolle). In Stoffwechselkäfigen wurde der 24 h-Harn zur Proteinbestimmung gesammelt.

Die mit Puromycin behandelten Tiere erhielten am 6., 7., 8. und 9. Versuchstag 200 mg/kg Orosomucoid i.v. oder analog isotone Kochsalzlösung (positive Kontrollen).

Am 10. Tag wurden die Tiere gewogen und durch Herzpunktion zur Plasmagewinnung getötet. Es wurde das Nierenfeuchtgewicht bestimmt und aus dem Plasma Kreatinin und Harnstoff gemessen.

Folgende Parameter wurden dabei ermittelt: Harn-Gesamteiweiß (mg/24 h), Plasmakreatinin (mg/dl), Blutharnstoff (mg/dl) und der Nierenindex (Nierengewicht in % des Körpergewichtes).

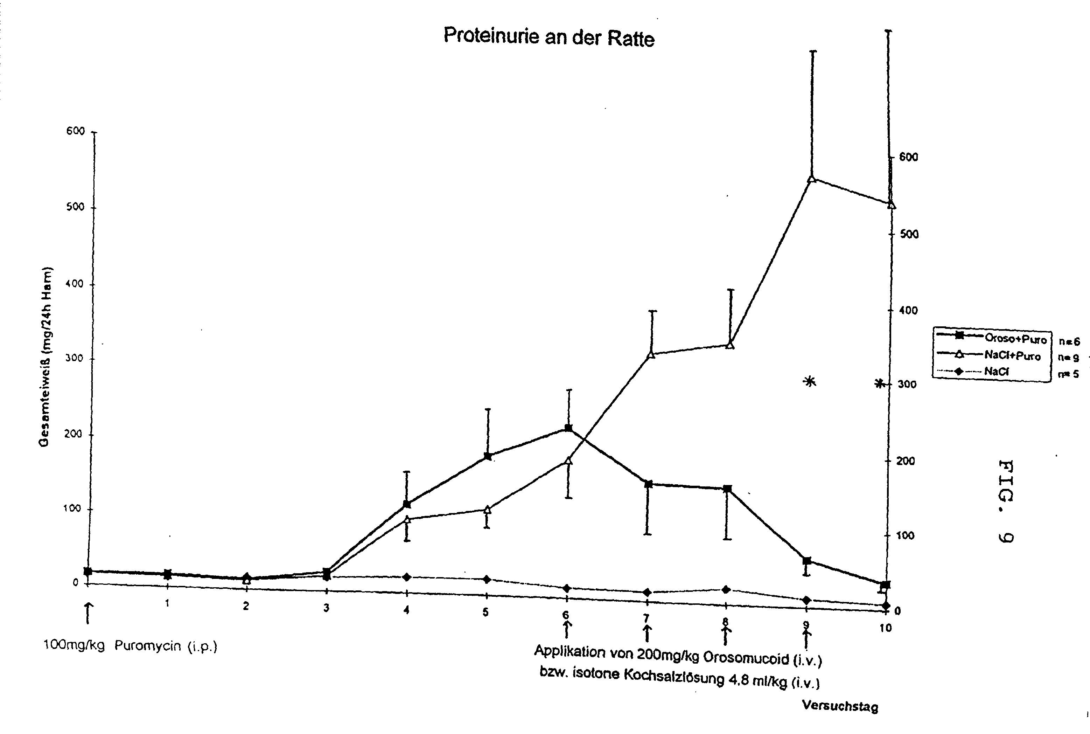

In Fig. 9 sind die Proteinurie-Werte des ersten Versuchsdurchganges dargestellt: Tiere, die am Tag 0 mit isotoner Kochsalzlösung behandelt worden waren, zeigten eine geringe, physiologische Proteinurie (volle Rauten). Bei Tieren, die mit Puromycin behandelt wurden, stieg das Eiweiß im Harn ab dem 3. Tag an. Bei Tieren, die an den Tagen 6 bis 9 isotone Kochsalzlösung erhielten, erreichte das Gesamteiweiß 500 bis 600 mg/24 h (offene Dreiecke). Bei Tieren, die an den Tagen 6 bis 9 mit Orosomucoid behandelt wurden, fiel die Proteinausscheidung praktisch auf Kontrollwerte ab (volle Quadrate).

## Patentansprüche

1. Verwendung von humanem α₁-sauren Glycoprotein zur Herstellung einer pharmazeutischen Präparation zur Behandlung von hämorrhagischem und/oder hypovolämischem Schock.

2. Verwendung nach Anspruch 1, **dadurch gekennzeichnet, dass** humanes α₁-saures Glycoprotein zur Herstellung einer Präparation zur Stabilisierung des intravasalen Volumens, insbesondere bei akuten Blutungen, exzessivem Plüssigkeitsverlust bzw. Vasidilationen, verwendet wird.

3. Verwendung nach Anspruch 1 oder 2, **dadurch gekennzeichnet, dass** die pharmazeutische Präparation als lagerstabile Infusionslösung hergestellt wird.

4. Verwendung nach einem der Ansprüche 1 bis 3, **dadurch gekennzeichnet, dass** die pharmazeutische Präparation als Lyophilisat hergestellt wird.

5. Verwendung nach einem der Ansprüche 1 bis 4, **dadurch gekennzeichnet, dass** die pharmazeutische Präparation in einer Dosis im Bereich von 70 mg/kg bis 5 g/kg vorliegt, insbesondere im Bereich von 100 bis 700 mg/kg.

6. Verwendung nach einem der Ansprüche 1 bis 5, **dadurch gekennzeichnet, dass** die pharmazeutische Präparation mindestens 50% α₁-saures Glycoprotein, insbesondere mehr als 70%, vorzugsweise mehr als 90%, bezogen auf das Gesamtprotein enthält.

7. Verwendung nach einem der Ansprüche 1 bis 6, **dadurch gekennzeichnet, dass** die pharmazeutische Präparation weiters Albumin enthält.

8. Verwendung nach einem der Ansprüche 1 bis 7, **dadurch gekennzeichnet, dass** die pharmazeutische Präparation einen Stabilisator enthält, insbesondere Natriumcaprylat.

9. Verwendung nach einem der Ansprüche 1 bis 8, **dadurch gekennzeichnet, dass** die pharmazeutische Präparation zur Inaktivierung bzw. Abreicherung von Viren behandelt ist, insbesondere durch mindestens eine physikalische Behandlung, wie Kitzebehandlung und/oder Filtration.

10. Verwendung nach einem der Ansprüche 1 bis 9, **dadurch gekennzeichnet, dass** die Präparation zusätzlich vasoaktive Substanzen, insbesondere Katecholamine, umfasst.

11. Verwendung nach Anspruch 10, **dadurch gekennzeichnet, dass** die vasoaktiven Substanzen gemeinsam oder parallel zu verabreichen sind.

12. Infusionspräparat zur Behandlung von hämorrhagischen und/oder hypovolämischen Schockzuständen enthaltend als wirksame Bestandteile α₁-saures Glycoprotein und eine vasoaktive Substanz.

13. Set zur Behandlung von hämorrhagischen und/oder hypovolämischen Schockzuständen umfassend
a) α₁-saures Glycoprotein in einer pharmazeutischen Präparation
und
b) eine vasoaktive Substanz.

## Claims

1. The use of human α₁-acid glycoprotein for producing a pharmaceutical preparation for treating hemorrhagic and/or hypovolemic shock.

2. The use according to claim 1, **characterised in that** human α₁-acid glycoprotein is used for producing a preparation for stabilizing the intravasal volume, in particular in case of acute hemorrhages, excessive loss of liquid or vasodilation, respectively.

3. The use according to claim 1 or 2, **characterised in that** the pharmaceutical preparation is produced as a storage-stable infusion solution.

4. The use according to any one of claims 1 to 3, **characterised in that** the pharmaceutical preparation is produced as a lyophilisate.

5. The use according to any one of claims 1 to 4, **characterised in that** the pharmaceutical preparation is present in a dose ranging from 70 mg/kg to 5 g/kg, in particular ranging from 100 to 700 mg/kg.

6. The use according to any one of claims 1 to 5, **characterised in that** the pharmaceutical preparation comprises at least 50% of α₁-acid glycoprotein, in particular more than 70%, preferably more than 90%, based on the total protein.

7. The use according to any one of claims 1 to 6, **characterised in that** the pharmaceutical preparation further comprises albumin.

8. The use according to any one of claims 1 to 7, **characterised in that** the pharmaceutical preparation comprises a stabilizer, in particular sodium caprylate.

9. The use according to any one of claims 1 to 8, **characterised in that** the pharmaceutical preparation is treated for virus inactivation or virus depletion, respectively, in particular by at least one physical treatment, such as heat treatment and/or filtration.

10. The use according to any one of claims 1 to 9, **characterised in that** the preparation additionally comprises vasoactive substances, in particular catechol amines.

11. The use according to claim 10, **characterised in that** the vasoactive substances are to be administered together or in parallel.

12. An infusion preparation for treating hemorrhagic and/or hypovolemic shock conditions, comprising α₁-acid glycoprotein and a vasoactive substance as its active components.

13. A kit for treating hemorrhagic and/or hypovolemic shock conditions, comprising
a) α₁-acid glycoprotein in a pharmaceutical preparation,
and
b) a vasoactive substance.

## Revendications

1. **Utilisation d'α1 glycoprot**éine acide humaine pour produire une préparation pharmaceutique destinée au traitement de choc hémorragique et / ou hypovolémique.

2. Utilisation selon la revendication 1, **caractérisée en ce que** l'**α1 glycoprot**éine acide humaine est utilisée pour produire une préparation destinée à stabiliser le volume intravasculaire, en particulier, en cas d'hémorragies aiguës, de pertes de liquide excessives ou de vasodilatations.

3. Utilisation selon la revendication 1 ou 2, **caractérisée en ce que** la préparation pharmaceutique est produite sous forme d'une solution pour perfusion stable lors du stockage.

4. Utilisation selon l'une quelconque des revendications 1 à 3, **caractérisée en ce que** la préparation pharmaceutique est produite sous forme de lyophilisat.

5. Utilisation selon l'une quelconque des revendications 1 à 4, **caractérisée en ce que** la préparation pharmaceutique se présente sous la forme d'une dose s'inscrivant dans une plage de 70 mg/kg à 5g/kg, et notamment dans une plage de 100 à 700 mg/kg.

6. Utilisation selon l'une quelconque des revendications 1 à 5, **caractérisée en ce que** la préparation pharmaceutique contient au moins 50% d'α**1** glycoprotéine acide, et en particulier, plus de 70 %, de préférence plus de 90 %, rapportée à la teneur en protéine globale.

7. Utilisation selon l'une quelconque des revendications 1 à 6, **caractérisée en ce que** la préparation pharmaceutique contient par ailleurs de l'albumine.

8. Utilisation selon l'une quelconque des revendications 1 à 7, **caractérisée en ce que** la préparation pharmaceutique contient un agent stabilisant, en particulier du caprylate de sodium.

9. Utilisation selon l'une quelconque des revendications 1 à 8, **caractérisée en ce que** la préparation pharmaceutique est utilisée pour inactiver ou affaiblir les virus, en particulier au moyen d'au moins un traitement physique, tel que le traitement thermique et / ou la filtration.

10. Utilisation selon l'une quelconque des revendications 1 à 9, **caractérisée en ce que** la préparation contient en plus des substances vasoactives, en particulier de la catécholamine.

11. Utilisation selon la revendication 10, **caractérisée en ce que** les substances vasoactives doivent être administrées parallèlement ou conjointement.

12. Préparation pour perfusion destinée à traiter les états de choc hémorragiques et / ou hypovolémiques **contenant comme principes actifs de l'α1 glycoprot**éine acide humaine et une substance vasoactive.

13. Ensemble pour le traitement des états de choc hémorragiques et / ou hypovolémiques comprenant
a) **de l'α1 glycoprot**éine acide humaine dans une préparation pharmaceutique
et
b) une substance vasoactive.
